# EUROPEAN PATENT APPLICATION

(11) **EP 4 338 753 A2**
(43) Date of publication of application: **20.03.2024**
(21) Application number: 24154347.9
(22) Date of filing: 02.03.2021
(51) Int. Cl.: A61K 47/44

(54) **FORMULATION COMPRISING CALCIUM SILICATE**

(30) Priority: 02.03.2020 SE 2030061; 25.08.2020 SE 2030267
(62) Divisional of application: 21710637.6
(71) Applicant: Lea Cares, 752 37 Uppsala (SE)
(72) Inventor: ENGQVIST, Håkan, 752 29 Uppsala (SE)
(74) Representative: Barker Brettell Sweden AB

(57) **Abstract**

Calcium and silicon are known to strengthen the formation of skin. The efficacy of oral intake of calcium and silicon is very difficult. Local delivery and uptake of the ions would enable more efficient strengthening of skin. The invention relates to the addition of calcium silicate to products for local release of calcium and silicate to skin.

## Description

### FIELD OF INVENTION

The invention relates to the field of formulations, especially formulations comprising calcium silicate for use in hair conditioners and/or nail hardeners.

### BACKGROUND

Our current life style has drastically increased the global prevalence of diabetes. In 2017 8.8 % of the global population was diagnosed with this chronic disease and the prevalence is expected to further increase to 9.9% by the year 2045 [1]. While considerable research and drug development efforts are in place to diminish the major risks related to being diagnosed with diabetes there are still problematic areas where adequate solutions are lacking. Diabetic patients often have a deteriorated nail standard since the breakdown of sugar can affect the building and quality of collagen in the human nail [2]. Patients often also have affected hair and skin. Today people with a deteriorated nail standard can take calcium and silicon (most commonly in the form of silicon dioxide) oral supplements to improve nail health. These elements are key ingredients in the nail building process [3, 4] While oral silicon and calcium have been proven to significantly enhance bone quality in osteoporosis patients [5], it is questionable if the oral route is the optimal one for delivery of these elements to nails of diabetes patients due to a reduced circulation to the nail matrix in such patients [6].

This invention relates to local delivery of calcium and silicon to the nail, skin or hair using traditional products as a delivery system, thus avoiding systemic administration and poor circulation preventing the elements from reaching their targets. A successful outcome could, hence, render nail products with a dual function as a delivery vehicle for therapeutic ions and as a beauty product. I Today's nail polish, containing a mix of organic polymers, pigments and other brand-specific components, is essentially used for decoration and protection of the nail plate [7, 8].

### SUMMARY

In one aspect of the invention there is a formulation, for use on nails, skin or hair, comprising calcium silicates for local release of calcium and silicon ions. In one embodiment of the invention there is a formulation which includes calcium silicate (CaOSiO₂), di calcium silicate ((CaO)₂SiO₂), tri calcium silicate ((CaO)₃SiO₂) or combinations thereof.

In one embodiment of the invention there is a formulation which includes 0.001 wt.% to 20 wt.%. preferably in concentrations 0.01 to 3 wt.% of calcium silicates.

In one embodiment of the invention there is a formulation which includes 0.01 to 3 wt.% calcium silicate (CaOSiO₂).

In one embodiment of the invention there is a which includes 0.01 to 3 wt.% di calcium silicate ((CaO)₂SiO₂).

In one embodiment of the invention there is a formulation, wherein the calcium silicate powder having a grain size of 10 nm to 300 micrometer.

In one embodiment of the invention the calcium silicate powder having a grain size of 10 nm to 50 micrometer.

In one embodiment of the invention the calcium silicate powder having a grain size of 10 nm to 25 micrometer.

In one embodiment of the invention the calcium silicate powder having a grain size of below 25 micrometer.

In one aspect of the invention there is a method of locally releasing calcium and silicon via incorporating calcium silicates in products to be applied on nails, skin or hair.

In one aspect of the invention there is a formulation comprising 0.01-3 wt% calcium silicate, wherein:
- the calcium silicate is in the form of a powder having grain size of 100 nm - 25 µm;
- the calcium silicate is in the form of a mixture of calcite, dicalcium silicate and tricalcium silicate, or in the form of wollastonite, or a mixture of those.

In one embodiment of the invention the calcium silicate is in the form of Portland cement.

In one embodiment of the invention the calcium silicate is in the form of Wollastonite.

In one embodiment of the invention the formulation according to the invention further comprises Fe.

In one aspect of the invention there is a nail hardener comprising a formulation according to the invention.

In one aspect of the invention there is a use of a nail hardener according to the invention for delivering calcium silicate to nails.

In one aspect of the invention there is a method of using a nail hardener according to the invention for application on nails.

In one aspect of the invention there is a hair conditioner comprising the composition according to the invention.

### FIGURES

**Figure 1** shows an X-ray diffractogram and a SEM image (inset) of one embodiment of the invention;
**Figure 2 a-d** shows photographs of one example of the invention;
**Figure 3** shows one example of the invention;
**Figure 4 a-c** shows SEM images of embodiments of the invention; and
**Figure 5** shows a SEM image of one embodiment of the invention.

### DETAILED DESCRIPTION

Ca-salts is often soluble in water. The solubility of calcium silicates (a Ca-salt) increases with the amount of Ca in the silicate. This invention aims to take advantage of the solubility of the calcium silicate to locally deliver Ca and silicate to nails, skin and hair.

The calcium silicate powder can be added to nail oils, nail polish, nail gels, nail creams and nail serums. The calcium silicate powder can be added to the nail formulations during formulation of the product using ordinary mixing equipment. The calcium silicate powder has to be distributed in the formulation for it to be well spread over the nail when applying the product. By adding a small ball to the container, the powder can be evenly distributed by shaking the container before application. The amount of added calcium silicate (by weight) is between 0.001 to 20 %, preferably 0.01 to 3%.

According to another part of the invention the calcium silicate powders, as described above, can also be added to hair and skin products, in concentrations of 0.001 wt.% to 20 wt.%, preferably in concentrations 0.01 to 3 wt.%. The calcium silicate powder can be added to the hair and skin formulations during formulation of the product using ordinary mixing equipment.

The following calcium silicate phases are suitable for the invention: calcium silicate (CaOSiO₂), di calcium silicate ((CaO)₂SiO₂) and tri calcium silicate ((CaO)₃SiO₂). The phases can be mixed into any variance. Such, calcium silicate salts are mildly soluble in water (or humid environment) and provide a source for slow release of the ions.

The grain size can vary between 10 nm to 300 micrometer, preferably between 10 nm and 50 micrometer, even more preferred 10 nm to 25 micrometer and most preferred 100 nm to 25 micrometer.

In one aspect of the invention there is a formulation comprising 0.01-3 wt% calcium silicate. The formulation can be oil-based, or water-based, or an emulsion. In one embodiment the formulation is oil-based. The formulation can be a nail polish, a nail oil, a skin lotion, a skin creme, or a hair conditioner. The calcium silicate is in the form of a powder having grain size of 100 nm - 25 µm. In one embodiment the calcium silicate is in the form of Portland cement (comprising calcite, dicalcium silicate, and tricalcium silicate). In one embodiment the calcium silicate is in the form of Wollastonite (CaOSiO₂). In one embodiment the calcium silicate is a mixture Wollastonite and Portland cement.

Portland cement can comprise elements in the form of trace elements, such as Cr, Fe, Cu, Ti, Mn, and Ni in concentrations of 10-2000 mg/kg. It is advantage that the final formulation comprises Fe, in a concentration of 1800- 2000 mg/kg.

In one embodiment the formulation is in the form of a nail hardener, that can be used to increase the hardness of nails.

In one embodiment the formulation is in the form of a hair conditioner that can be used to improve the hair quality.

In one aspect of the invention there is a use of a nail hardener for delivering of calcium silicate to nails. In one aspect of the invention there is a use of a hair conditioner for delivering calcium silicate to hair.

In one aspect of the invention there is a method for using a nail hardener for application on nails. After application of a nail hardener according to the invention the quality of the nails can be improved. In one aspect of the invention there is a method for using a hair conditioner for treatment of hair. After a treatment of hair using a hair conditioner according to the invention the quality of the hair can be improved.

All aspects and embodiments can be combined with each other.

### EXAMPLES

### 1. Example for nail polish

### Materials and Methods

### Materials

*Nail polish:* To form the basis for a delivery system that simultaneously may function as a beauty product we use the commercial base coat nail polish 7 *Day Base Coat* (item number 1092- 164-0000, Depend Cosmetics AB, Halmstad, Sweden) as carrier of calcium and silicon. Base coats are used to prevent discoloration from pigmented nail polish and to give the nail a smooth surface [9]. They are usually denoted as Step 2 layers (to be applied after a Step 1 cleanser procedure). Since the base coat is applied as the innermost layer in direct contact with the nail, the functional elements will not have to pass a barrier nail polish layer as would be the case if a pigmented, so-called Step 3 layer, or a top coat (Step 4 layer) was to be utilized as delivery vehicle.

*Calcium and silicon source:* 1. White Portland Cement (WPC, Aalborg Portland A/S, Aalborg, Denmark), containing calcite (CaCO), dicalcium silicate (2CaO•SiO₂) and tricalcium silicate (3CaO•Si₂), was used as a source of calcium and silicon. 2. Pure calcium silicate (Wollastonite, CaOSiO₂) was also used as a source of calcium and silicon in a second group.

Artificial (testing substrate) nails (Vitro-nails^{®}, IMC Inc, Florida, USA) as well as human nails (donated by one of the authors) were employed to analyse the presence of Ca and Si after application of nail polish.

### Preparation and analysis of the White Portland Cement

Sieving: The as-delivered White Portland Cement (WPC) was sieved (Retsch Vibratory Sieve Shaker AS 200 Basic, Retsch GMBH, Haan, Germany) to obtain a particle size < 25 µm to allow for optimal dispersion in the base coat. The 2CaO•SiO₂ was used as received with a grain size of below 25 µm.

Elemental analysis: The amount of Ca, Si, Cr, Mn, Fe, Cu, Ti, Ni in the powders was analysed by Inductively Coupled Plasma Optical Emission Spectrometry (ICP-OES, Avio 200 Perkin Elmer, Waltham, MA, USA) by using commercial calibration standards (Perkin Elmer) monitored at the emission lines 317.933 nm, 251.611 nm, 283.563 nm, 257.610 nm, 238.204 nm, 324.752 nm, 368.519 nm, 341.476 nm, respectively. Testing samples (1 mg/mL) were prepared by dissolving the sieved powder into 2 vol % HNO₃ for 24 h at 37 °C. The samples were thereafter filtered (0.45pm) and diluted with MilliQ water (QPAK 2, Millipore) to appropriate testing concentrations. A precision test was automatically done in Syngistix for ICP-OES (PerkinElmer, Inc.; version 2.0). The relative standard deviation (RSD) value, reported as a percentage, as it is indicative of the short-term precision of several measurements for a strong emission line. Scanning Electron Microscopy (SEM): SEM images of the powders were obtained on a TM-1000 tabletop SEM (Hitachi, Chiyoda, Japan), with a solid-state back- scatter electron detector, acceleration voltage of 15 keV, emission current of 195 µA or 52 µA and a working distance of 7 mm. The samples were prepared by mounting the powder on SEM stubs using a double-sided adhesive carbon tape and excess powder was removed using a compressed air gun. X-Ray Diffraction (XRD): The phase composition of the powder was investigated by XRD with a D5000 (Siemens) with Cu Ka radiation (λ = 1.5418 Å) using a step size of 0.050°.

### Preparation and analysis of nail polish formulations

Formulation preparation: Six nail polish formulations containing WPC were prepared by adding 5.2 mg, 10.0 mg, and 15.0 mg, respectively, of the Wollastonite and sieved WPC into 5 mL of nail polish (equal about 0. 1-0.3 wt.%). For simplicity the nail polish formulations were called np 1-6, respectively. The samples were homogenized by shaking with two zirconia beads (5 mm) in each bottle, to ensure a homogeneous powder distribution. SEM analysis of formulations: Thin layers of each sample nail polish were painted on a plastic surface and left to dry overnight. Upon drying, the samples were examined by SEM to obtain information about the WPC particle distribution in the dry polish.

### Elemental release from nail polish

Release into MilliQ water: An area of 1.0×1.5 cm² of each sample nail polish was painted on a plastic surface and left to dry overnight. The dried nail polish samples were then carefully weighed and adjusted to ensure that the same amount of nail polish was placed in separate centrifuge tubes filled with 50 mL of MilliQ water. The surface area-to-volume ratio of water was set to 1.5 cm² /mL, according to ISO Standards 10993-12:2002 and 10993-15:2000 [10, 11]. The centrifuge tubes were additionally covered with Parafilm to prevent evaporation and stored in an oven at 37 °C. The calcium and silicon contents in the MilliQ water were analyzed by ICP-OES after 4 and 48 h. Release onto artificial and human nails: Artificial nails were painted with a ~ 1.5 mm thick layer of the three different nail polish formulations (np 1-3). A human nail was painted with np3. All nail samples were stored in a 37 °C oven during 120 h. The nail polish was thereafter removed with acetone and the nails were examined by SEM. Artificial and human nails painted with np3 were analyzed by dispersive X-ray Fluorescence Spectroscopy (EDX) using an acquisition time of 120 s, a process time of 4 s, and an acceleration voltage of 15.0 kV.

### Results

### Analysis of the WPC and Wollastonite

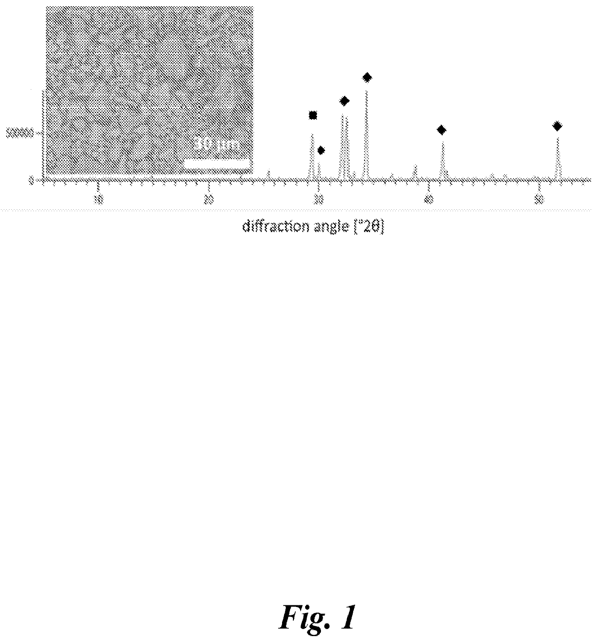

The elemental analysis performed on the sieved WPC showed that in addition to calcium and silicon, which are inherent to the WPC, the material also contained Cr (14.0 mg/kg), Fe (1962.0 mg/kg), Cu (22.9 mg/kg), Ti (458.0 mg/kg), Mn (131.0 mg/kg) and Ni (48.5 mg/kg). To the best of our knowledge, none of the observed trace elements have been found to be harmful for nails in low doses while one of them, iron, on the other hand, is known to have beneficial effects [12]. Figure 1 shows an X-ray diffractogram and a SEM image (inset) of the sieved WPC used as calcium and silicon source in the base coat nail polish formulation. In Figure 1 (■) CC = calcite; (•) C₂S = dicalcium silicate; and (◆) C₃S = tricalcium silicate.

We observe that the particle size of the sieved powder is below 25 µm, as intended. As expected [13], the material was composed of calcite (CC) as well as dicalcium silicate (C₂S) and tricalcium silicate (C₃S). The Wollastonite samples did not contain any trace elements.

### Analysis of the Nail Polish Formulations

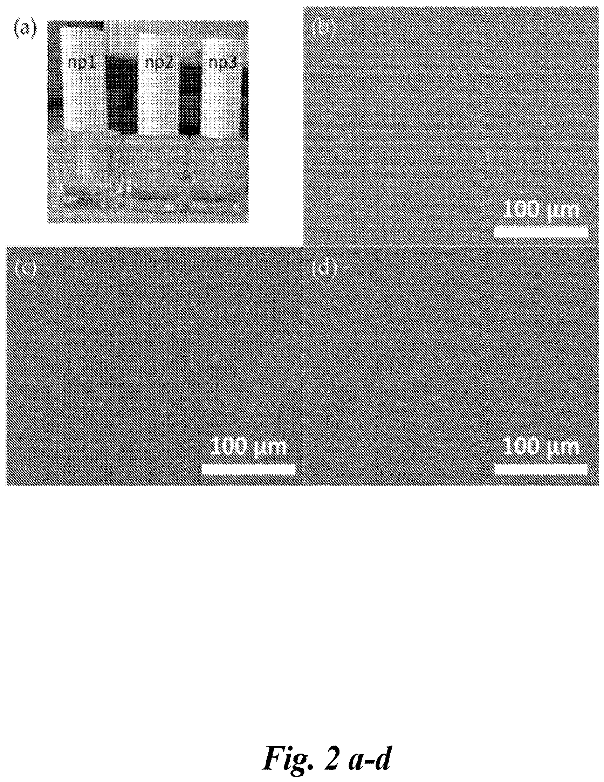

Figure 2 shows a photography of the three nail polish formulations with WPC along with SEM images of painted and dried layers of them. In Figure 2, (a) shows a photograph of the modified nail polish formulations, and (b), (c), and (d) shows SEM micrographs of the dry nail polish formulations: (b) np1, (c) np2, (d) np3. The SEM micrographs show that the nail polish formulations become increasingly opaque with increasing WPC content indicating that the particle density in the painted layers increases correspondingly. Whereas no particles of ~25 µm in size are observed in the SEM images, all SEM images in Figure 2 show that the particles are not fully dissolved in the formulations. This is expected since an excess of water would have been needed to allow all particles to fully dissolve.

### Calcium and Silicon Release from Nail Polish Formulations

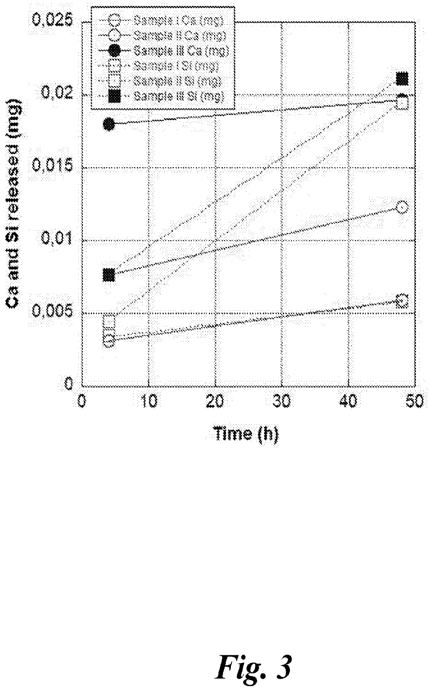

Figure 3 shows the amount of calcium and silicon released into MilliQ water from the three nail polish formulations (np1, np2 and np3) as detected using ICP-OES. The results show that for both elements an increasing amount is released with increasing amount of WPC in the formulations. It is also observed that the amount released after 48 h is higher than the amount released after 4 h for all samples and both elements. Whereas the increase in released amount as a function of time is quite moderate for calcium, the released amount of silicon is 3-4 times higher after 48 h as compared to 4 h for np2 and np3. Sample np1 was shown to release a very similar amount of calcium and silicon at both analyzed time points. It is noticeable that during the initial release (4 h) from the two samples with the highest WPC content, calcium is dominating while after a prolonged release (48 h) the amount of silicon released from all samples is similar or higher than the corresponding amount of calcium (up to 1.6 times higher for np3). The latter is interesting bearing in mind that the Ca/Si ratio of WPC significantly exceeds 1 both in molar and weight units.

It should be noted that the dried nail polish immersed into the MilliQ water contained -0.2-0.5 mg of WPC so the released amount of both elements under study is of the order of 1 % of the corresponding elemental content in the nail polish layer. The results for np4, np5 and np 6 also showed release of calcium and silicon as function of time. Figure 3 shows the total amount of calcium and silicon released from dried nail polish layers into 50 mL of MilliQ water as obtained from ICP-OES analysis. The relative standard deviations were less than 2% for all data points.

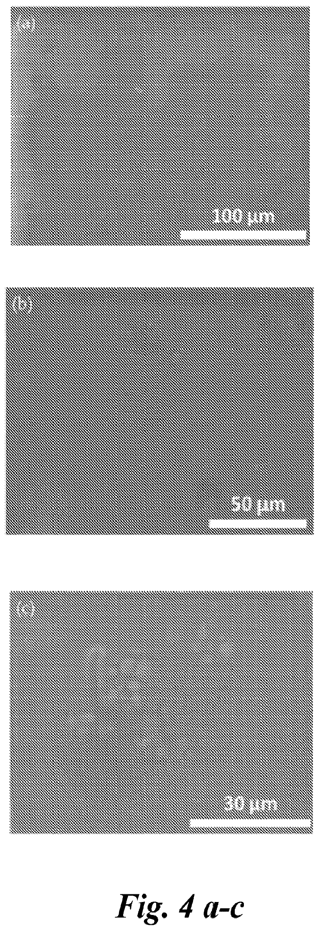

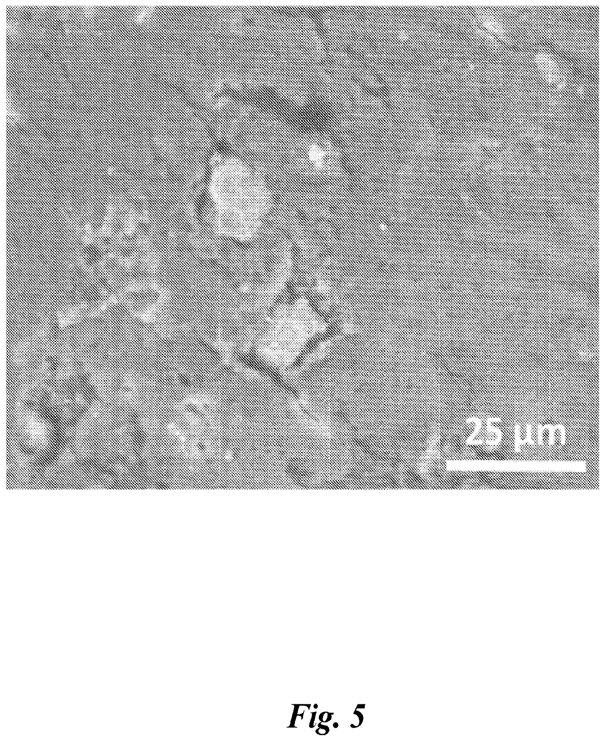

Figure 4 shows SEM images of the artificial nails after having been coated with the different nail polish formulations for 5 days. Figure 4 shows SEM micrographs of artificial nails after having been coated with (a) np1, (b) np2, (c) np3 for 5 days. An increasing amount of particles can be observed with increasing WPC in the formulations. The EDX analysis verified that the particles observed in the artificial nail that had been exposed to np3 (bottom image) indeed contained calcium and silicon; an analysis of an area containing several particles found an elemental content of A1 (54.5 wt%), Si (32.3 wt%), Ca (9.1 wt%) and other elements (4.0 wt%). It can, hence, be concluded that calcium and silicon from the nail polish enter the artificial nail. Figure 5 shows a SEM micrograph of human nail after having been coated with np3 for 120 h. The scale bar is 25 µm.

### Conclusions

It is found that delivery of both elements takes place to both artificial and human nails.

### 2. Example nail oil

Wollastonite - CaOSiO₂ and White Portland Cement with a grain size below 25 micrometer was mixed into a nail oil formulation.

The nail oil used was Depend nail care Myrra & Avocado oil. 0.1 wt.% weight percent and 2 wt.% of calcium silicate was added to the oil. The release of Ca and Silicate was tested in water. A droplet of the calcium silicate containing oil was add into separate centrifuge tubes filled with 50 mL of MilliQ water. The centrifuge tubes were additionally covered with Parafilm to prevent evaporation and stored in an oven at 37 °C. The calcium and silicon contents in the MilliQ water were analyzed by ICP-OES after 4 and 48 h.

White Portland Cement (WPC, Aalborg Portland A/S, Aalborg, Denmark), containing calcite (CaCOs), dicalcium silicate (2CaO•SiO₂) and tricalcium silicate (3CaO•SiO₂), was used as one source of calcium and silicon. The as-delivered White Portland Cement (WPC) was sieved (Retsch Vibratory Sieve Shaker AS 200 Basic, Retsch GMBH, Haan, Germany) to obtain a particle size < 25 µm to allow for optimal dispersion. Elemental analysis: The amount of Ca, Si, Cr, Mn, Fe, Cu, Ti, Ni in the sieved powder was analysed by Inductively Coupled Plasma Optical Emission Spectrometry (ICP-OES, Avio 200 Perkin Elmer, Waltham, MA, USA) by using commercial calibration standards (Perkin Elmer) monitored at the emission lines 317.933 nm, 251.611 nm, 283.563 nm, 257.610 nm, 238.204 nm, 324.752 nm, 368.519 nm, 341.476 nm, respectively. Testing samples (1 mg/mL) were prepared by dissolving the sieved powder into 2 vol % HNO₃ for 24 h at 37 °C. The samples were thereafter filtered (0.45 µm) and diluted with MilliQ water (QPAK 2, Millipore) to appropriate testing concentrations. A precision test was automatically done in Syngistix for ICP-OES (PerkinElmer, Inc.; version 2.0). The relative standard deviation (RSD) value, reported as a percentage, as it is indicative of the short term precision of several measurements for a strong emission line. Scanning Electron Microscopy (SEM): SEM images of the sieved powder were obtained on aTM-1000 tabletop SEM (Hitachi, Chiyoda, Japan), with a solid-state back- scatter electron detector, acceleration voltage of 15 keV, emission current of 195 µA or 52 µA and a working distance of 7 mm. The samples were prepared by mounting the powder on SEM stubs using a double-sided adhesive carbon tape and excess powder was removed using a compressed air gun. X-Ray Diffraction (XRD): The phase composition of the powder was investigated by XRD with a D5000 (Siemens) with Cu Ka radiation (λ = 1.5418 Å) using a step size of 0.050°.

Wollastonite powder was sieved (Retsch Vibratory Sieve Shaker AS 200 Basic, Retsch GMBH, Haan, Germany) to obtain a particle size < 25 µm to allow for optimal dispersion.

The results showed release of both elements from the formulations. The WPC showed higher release than the Wollastonite powder. It was also observed that the amount released after 48 h was higher than the amount released after 4 h for all samples and both elements.

### 3. Example skin creme

Wollastonite - CaOSiO₂ with a grain size below 25 micrometer was mixed into a skin lotion formulation. The skin lotion used was Nivea Creme - body lotion and 0.5 wt.% of calcium silicate was added. Wollastonite powder was sieved (Retsch Vibratory Sieve Shaker AS 200 Basic, Retsch GMBH, Haan, Germany) to obtain a particle size < 25 µm to allow for optimal dispersion.

The release of Ca and Silicate was tested in water. A droplet of the calcium silicate containing Creme was add into separate centrifuge tubes filled with 50 mL of MilliQ water. The centrifuge tubes were additionally covered with Parafilm to prevent evaporation and stored in an oven at 37 °C. The calcium and silicon contents in the MilliQ water were analyzed by ICP- OES after 4 and 48 h.

The results showed release of both elements from the formulation. It was also observed that the amount released after 48 h was higher than the amount released after 4 h for all samples and both elements.

### 4. Example hair conditioner

Wollastonite - CaOSiO₂ with a grain size below 25 micrometer was mixed into a hair conditioner formulation.

The hair conditioner used was Nivea Hair milk Conditioner and 0.5 wt.% of calcium silicate was mixed into the hair conditioner. Wollastonite powder was sieved (Retsch Vibratory Sieve Shaker AS 200 Basic, Retsch GMBH, Haan, Germany) to obtain a particle size < 25 µm to allow for optimal dispersion.

The release of Ca and Silicate was tested in water. A droplet of the calcium silicate containing hair conditioner was add into separate centrifuge tubes filled with 50 mL of MilliQ water. The centrifuge tubes were additionally covered with Parafilm to prevent evaporation and stored in an oven at 37 °C. The calcium and silicon contents in the MilliQ water were analyzed by ICP-OES after 4 and 48 h. The results showed release of both elements from the formulation. It was also observed that the amount released after 48 h was higher than the amount released after 4 h for all samples and both elements.

### References

1. International Diabetes Federation. IDF Diabetes Atlas. 9th ed.; Brussels, Belgium: International Diabetes Federation, 2019.
2. Bristow, I. Non-ulcerative skin pathologies of the diabetic foot. Diabetes-Metab Res 2008, 24, S84-S89, doi: 10. 1002/dmrr.818.
3. Pravina, P.; Sayaji, D.; Avinash, M. Calcium and its role in human body. International Journal of Research in Pharmaceutical and Biomedical Sciences 2013, 4, 659-668.
4. Araujo, L.; Addor, F.; Campos, P.M.B.G.M. Use of silicon for skin and hair care: an approach of chemical forms available and efficacy. An Bras Dermatol 2016, 91, 331-335.
5. Jugdaohsingh, R. Silicon and bone health. J Nutr Health Aging 2007, 11, 99-110.
6. Hillson, R. Nails in diabetes. Practical Diabetes 2017, 34, 230-231, doi: 10. 1002/pdi.2124.
7. Chaundri, S.K.; Jain, N.K. History of cosmetics. Asian Journal of Pharmaceutics 2009, 3, 164-167.
8. Andre, J.; Baran, R. Nail Cosmetics: Handle of skin care. In Handbook of Cosmetic Science and Technology, 3rd edition ed.; Bare, A.O., Paye, M., Maibach, H.I., Eds. CRC Press: 2014; pp. 745-767.
9. lorizzo, M.; Piraccini, B.M.; Tosti, A. Nail cosmetics in nail disorders. J Cosmet Dermatol 2007, 6, 53-58, doi: 10. 1111 /j. 1473-2165.2007.00290.x.
10. Standardization, I.O.f. ISO 10993: Biological Evaluation of Medical Devices. In Part 12: Sample Preparation and Reference materials, 2002.
11. Standardization, I.O.f. ISO 10993: Biological Evaluation of Medical Devices. In Part 15: Identification and Quantification of Degradation Products From Metals and Alloys, 2000.
12. Seshadri, D.; De, D. Nails in nutritional deficiencies. Indian J Dermatol Ve 2012, 78, 237-241, doi: 10.4103/0378-6323.95437.
13. Stutzman, P.E.; Feng, P.; Bullard, J.W. Phase Analysis of Portland Cements by Combined Quantitative X-Ray Powder Diffraction and Scanning Electron Microscopy. J Res Natl Inst Stan 2016, 121, 47-107**.**

## Claims

1. A skin formulation, for use on skin comprising 0.01 to 3 wt.% of calcium silicate powder having a grain size of 10 nm to 300 micrometer, wherein the skin formulation is for local release of calcium and silicon ions to the skin.

2. The skin formulation as set forth in claim 1, wherein the calcium silicate powder includes calcium silicate (CaOSiO₂), di calcium silicate ((CaO)₂SiO₂), tri calcium silicate ((CaO)₃SiO2) or combinations thereof.

3. The skin formulation as set forth in claim 1 which includes 0.01 to 3 wt.% calcium silicate (CaOSiO₂).

4. The skin formulation as set forth in claim 1 which includes 0.01 to 3 wt.% di calcium silicate ((CaO)₂SiO₂).

5. The skin formulation as set forth in claim 1, wherein the skin formulation is oil-based.

6. The skin formulation as set forth in claim 1, wherein the skin formulation is a skin lotion.

7. The skin formulation as set forth in claim 1, wherein the skin formulation is a skin creme.

8. The skin formulation of claim 1, wherein the calcium silicate powder having a grain size of 10 nm to 50 micrometer.

9. The skin formulation of claim 1, wherein the calcium silicate powder having a grain size of 10 nm to 25 micrometer.

10. The skin formulation according to claim 1, wherein the calcium silicate powder is in the form of Portland cement powder.

11. The skin formulation according to claim 1, wherein the calcium silicate powder is in the form of Wollastonite powder.

12. The formulation according to claim 1, further comprising Fe.
